# EUROPÄISCHE PATENTSCHRIFT

(11) **EP 3 054 771 B1**
(45) Veröffentlichungstag und Bekanntmachung des Hinweises auf die Patenterteilung: **07.03.2018**
(21) Anmeldenummer: 14786445.8
(22) Anmeldetag: 06.10.2014
(51) Int. Cl.: A01N 43/90, C07C 43/205, A01N 25/30

(54) **VERETHERTE TRIBUTYLPHENOLALKOXYLATE, VERFAHREN ZU DEREN HERSTELLUNG UND DEREN VERWENDUNG IN PFLANZENSCHUTZMITTELN**
ETHERIFIED TRI-BUTYLPHENOL ALKOXYLATES, PROCESS FOR THEIR PREPARATION AND THEIR USE IN CROP PROTECTION AGENTS
ALKOXYLATES DE TRIBUTYLPHÉNOL ÉTHÉRIFIÉS, PROCÉDÉ DE PRODUCTION ET UTILISATION DESDITS ALKOXYLATES DE TRIBUTYLPHÉNOL ÉTHÉRIFIÉS DANS DES AGENTS PHYTOPROTECTEURS

(30) Priorität: 07.10.2013 EP 13187604
(43) Veröffentlichungstag der Anmeldung: 17.08.2016
(73) Patentinhaber: Clariant International Ltd, 4132 Muttenz (CH)
(72) Erfinder: BAUR, Peter, 86938 Schondorf (DE); MILBRADT, Robert, 65191 Wiesbaden (DE); SCHROEDER, Kai, 84489 Burghausen (DE); SCHWEINITZER, Gerd, 65934 Frankfurt am Main (DE)
(74) Vertreter: Paczkowski, Marcus
(86) Internationale Anmeldenummer: PCT/EP2014/002702
(87) Internationale Veröffentlichungsnummer: WO 2015/051899

(56) Entgegenhaltungen:
- WO-A1-2004/006671
- DE-A1- 10 258 856

## Beschreibung

Die Erfindung betrifft maskierte, d. h. veretherte Tributylphenolalkoxylate, Verfahren zu deren Herstellung, deren Verwendung zur Verbesserung der Wirkung von agrochemischen Wirkstoffen in und auf Pflanzen und Pflanzenschutzmittel, welche die veretherten Tributylphenolalkoxylate enthalten.
Die Verwendung von Tributylphenolalkoxylaten als Additive für Pflanzenschutzmittel ist bekannt. Nicht beschrieben wurde aber bisher eine Anwendung von an der Hydroxygruppe veretherten Tributylphenolalkoxylaten in Pflanzenschutzmitteln. DE 102 58 856 beschreibt Tributylpholalkoxylate als Additive für Pflanzenschutzmitteln. Es wurde nun gefunden, dass die Wirkung von Pflanzenschutzmitteln durch bestimmte, veretherte Tributylphenolalkoxylate verbessert wird. So fördern die erfindungsgemäßen veretherten Tributylphenolalkoxylate sowohl den Verbleib der Pflanzenschutzmittel auf der Pflanze, vor allem auf den Blättern (verbesserte Retention), als auch das Eindringen der in den Pflanzenschutzmitteln enthaltenen agrochemischen Wirkstoffe in die Pflanze (verbesserte Penetration). Diese Verbesserung der Eigenschaften wird bereits bei niedrigen Konzentrationen von veretherten Tributylphenolalkoxylaten erreicht.
Gegenstand der Erfindung sind daher veretherte 2,4,6-Tri-(2-butyl)phenolalkoxylate der Fomel (I), 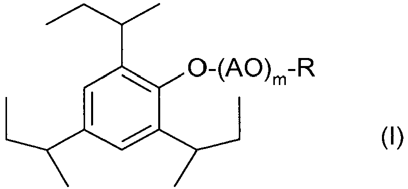 worin
- R: für Methyl steht, und
- A: für einen 1,2-Ethylen-Rest, einen 1,2-Propylen-Rest, einen 1,2-Butylen-Rest oder für Gemische aus Ethylen- und Propylen-Resten oder für Gemische aus 1,2-Ethylen- und 1,2-Butylen-Resten steht und
- m: für eine Zahl von 2 bis 30 steht.
Weiterhin Gegenstand der Erfindung ist ein Verfahren zur Herstellung der veretherten Tributylphenolalkoxylate, umfassend den Schritt
a) Umsetzung eines Tributylphenolalkoxylats der Formel (II), 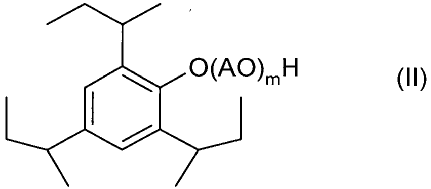 worin A und m die oben angegebenen Bedeutungen haben, mit einem C1-Alkylhalogenid unter Basenkatalyse.
Ebenso Gegenstand der Erfindung ist die Verwendung der veretherten Tributylphenolalkoxylate der Formel (I) in Pflanzenschutzmitteln, insbesondere zur Verbesserung der Wirkung von agrochemischen Wirkstoffen in und auf Pflanzen.
Ebenfalls Gegenstand der Erfindung ist ein Pflanzenschutzmittel, enthaltend
A) einen oder mehrere agrochemische Wirkstoffe und
B) ein oder mehrere veretherten Tributylphenolalkoxylate der Formel (I).
Weiterhin Gegenstand der Erfindung ist die Verwendung der veretherten Tributylphenolalkoxylate der Formel (I) als Tensid, als Netz und Haftmittel oder als Emulgator.

Die erfindungsgemäßen veretherten Tributylphenolalkoxylate sind durch die Formel (I) allgemein definiert. Bevorzugte Restedefinitionen der vorstehenden und nachfolgend genannten Formeln sind im Folgenden angegeben:
Bevorzugt haben die Symbole und Indizes in der Formel (I) folgende Bedeutungen:
   - R: ist Methyl

   - A: ist bevorzugt ein 1,2-Ethylen-Rest, ein 1,2-Propylen-Rest oder ein Gemisch aus 1,2-Ethylen- und 1,2-Propylen-Resten.
   - m: ist bevorzugt eine Zahl von 2 bis 20.
Besonders bevorzugt haben die Symbole und Indizes in der Formel (I) folgende Bedeutungen:
- R: ist Methyl.
- A: ist besonders bevorzugt ein 1,2-Ethylen-Rest.
- m: ist besonders bevorzugt eine Zahl von 3 bis 10.

Weiterhin ganz besonders bevorzugt ist m eine Zahl von 5 bis 8.
Die erfindungsgemäßen veretherten Tributylphenolalkoxylate können nach bekannten, dem Fachmann geläufigen Verfahren hergestellt werden. Dazu werden Tributylphenolalkoxylate der Formel (II), 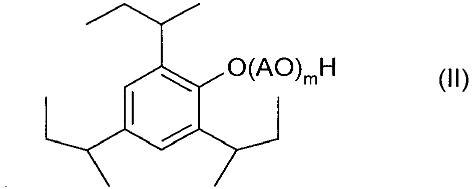 worin A und m die oben angegebenen Bedeutungen haben, mit einem C1-Alkylhalogenid bevorzugt einem Alkylchlorid, unter Basenkatalyse, bevorzugt mit NaOH, umgesetzt.
Die als Vorstufe eingesetzten Tributylphylalkoxylate der Formel (II) sind kommerziell erhältlich, beispielsweise unter den Sapogenat-Marken von Clariant. Sie können aus dem entsprechenden Phenol durch Umsetzung mit Alkylenoxiden (EO, PO, BO oder Gemischen daraus) unter Ringöffnung in Gegenwart von Katalysatoren hergestellt werden. Die Verfahrensbedingungen, der Verfahrensablauf und der Katalysator sind dem Fachmann bekannt. Gegenstand der Erfindung ist auch die Verwendung der erfindungsgemäßen veretherten Tributylphenolalkoxylate der Formel (I), 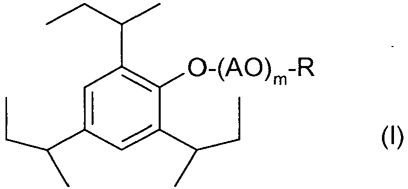 worin die Symbole R, A und m die oben angegebene Bedeutung haben zur Verbesserung der Wirkung von agrochemischen Wirkstoffen in und auf Pflanzen. Besonders bevorzugt ist dabei die Verbesserung der Penetration von agrochemischen Wirkstoffen in Pflanzen und die Verbesserung der Retention von agrochemischen Wirkstoffen an Pflanzen, insbesondere an Blättern. Die in der Literatur (Baur et al., 1997, Pesticide Science 51, 131-152) beschriebene Methode kann zur Bestimmung dieser Eigenschaften eingesetzt werden.
Gegenstand der Erfindung ist auch die Verwendung der erfindungsgemäßen veretherten Tributylphenolalkoxylate als Tensid, als Netz- und Haftmittel sowie als Emulgator.

Die Verbindungen der Formel (I) werden einzeln oder in Form von Gemischen eingesetzt. Wird in der Beschreibung oder den Ansprüchen von veretherten Tributylphenolalkoxylaten gesprochen, so sind ausdrücklich einzelne erfindungsgemäße Verbindungen oder Mischungen von mehreren erfindungsgemäßen Verbindungen gemeint.

Die Menge an einer oder mehreren Verbindungen der Formel (I) bei der erfindungsgemäßen Verwendung in Pflanzenschutzmitteln kann nach Wirkstoff und Formulierungstyp in weiten Grenzen variieren. Die Verbindungen der Formel (I) können in allen üblichen agrochemischen Formulierungen eingesetzt werden, bevorzugt in flüssigen. Gegenstand der Erfindung ist auch die Verwendung der veretherten Tributylphenolalkoxylate der Formel (I) zur Verbesserung der Wirkung auf Ebene der Pflanze als Tank-Mix-Additiv, d. h. dass die veretherten Tributylphenolalkoxylate erst direkt vor dem Ausbringen einer aus einer konzentrierten Formulierung hergestellten Spritzbrühe zugesetzt werden. Prinzipiell können die Verbindungen auch in feste Formulierungen eingebracht werden.

Die erfindungsgemäße Verwendung der veretherten Tributylphenolalkoxylate der Formel (I) erfolgt beispielsweise in anwendungsfertigen Pflanzenschutzmitteln (Spritzbrühen), in denen der Gehalt an einem oder mehreren veretherten Tributylphenolalkoxylaten der Formel (I)
- 0,01 bis 3 Gew.-%,
- besonders bevorzugt 0,01 bis 1 Gew.-%,
- ganz besonders bevorzugt 0,02 bis 0,5 Gew.-%,
- insbesondere bevorzugt 0,03 bis 0,3 Gew.-%
beträgt.

Enthält ein Pflanzenschutzmittel mehrere veretherten Tributylphenolalkoxylate, so ist die Mengenangabe als Gesamtgehalt aller veretherten Tributylphenolalkoxylate zu verstehen.

Die oben aufgeführten allgemeinen oder in Vorzugsbereichen aufgeführten Restedefinitionen, Wertebereiche bzw. Erläuterungen können auch untereinander, also zwischen den jeweiligen Bereichen und Vorzugsbereichen beliebig kombiniert werden.

Da der Wirkmechanismus der veretherten Tributylphenolalkoxylate als Penetrationsförderer grundsätzlich unabhängig von der Art des eingesetzten agrochemischen Wirkstoffs ist, kommt ihre Verwendung in allen Pflanzenschutzmitteln in Frage, die mindestens einen agrochemischen Wirkstoff enthalten, dessen biologische Wirksamkeit durch erhöhtes Eindringen in eine Kultur- oder Schadpflanze erhöht werden kann.

Da auch der Wirkmechanismus der veretherten Tributylphenolalkoxylate als Retentionsförderer grundsätzlich unabhängig von der Art des eingesetzten agrochemischen Wirkstoffs ist, kommt ihre Verwendung in allen Pflanzenschutzmitteln in Frage, die mindestens einen agrochemischen Wirkstoff enthalten, dessen biologische Wirksamkeit durch verbesserte Retention auf einer Kultur- oder Schadpflanze erhöht werden kann.

Unter "agrochemischen Wirkstoffen" werden im Rahmen der Erfindung Herbizide, Fungizide, Bakterizide, Insektizide, Akarizide, Molluskizide, Nematizide, Rodentizide Repellents sowie Phytohormone verstanden. Vorzugsweise genannt seien Fungizide, Bakterizide, Insektizide, Akarizide, Nematizide, Herbizide, Pflanzenwuchsregulatoren, Pflanzennährstoffe und Repellents. Die agrochemischen Wirkstoffe der erfindungsgemäßen Pflanzenschutzmittel werden vorzugsweise aus Herbiziden, Insektiziden, Akariziden und Fungiziden gewählt.

Eine Übersicht der wichtigsten agrochemischen Wirkstoffe findet sich beispielsweise in "The Pesticide Manual" des British Crop Protection Council, 16th Edition 2012, Editor: C D S Tomlin.

Bevorzugte Fungizide sind aliphatische Stickstofffungizide, Amidfungizide wie Acylaminosäurefungizide oder Anilidfungizide oder Benzamidfungizide oder Strobilurinfungizide, aromatische Fungizide, Benzimidazolfungizide, Benzothiazolfungizide, Carbamatfungizide, Conazolfungizide wie Imidazole oder Triazole, Dicarboximidfungizide, Dithiocarbamatfungizide, Imidazolfungizide, Morpholinfungizide, Oxazolfungizide, Pyrazolfungizide, Pyridinfungizide, Pyrimidinfungizide, Pyrrolfungizide, Chinonfungizide.

Bevorzugte Herbizide sind Amidherbizide, Anilidherbizide, aromatische Säureherbizide wie Benzoesäureherbizide oder Picolinisäureherbizide, Benzoylcyclohexanedionherbizide, Benzofuranylalkylsulfonatherbizide, Benzothiazolherbizide, Carbamatherbizide, Carbanilatherbizide, Cyclohexenoximherbizide, Cyclopropylisoxazolherbizide, Dicarboximidherbizide, Dinitroanilinherbizide, Dinitrophenolherbizide, Diphenyletherherbizide, Dithiocarbamatherbizide, Imidazolinonherbizide, Isoxazolidinonherbizide Nitrilherbizide, Organophosphorherbizide, Oxadiazolonherbizide, Oxazolherbizide, Phenoxyherbizide wie Phenoxyessigsäureherbizide oder Phenoxybutansäureherbizide oder Phenoxypropionsäureherbizide oder Aryloxyphenoxypropiosäureherbizide, Pyrazolherbizide wie Benzoylpyrazolherbizide oder Phenylpyrazolherbizide, Pyridazinonherbizide, Pyridinherbizide, Thiocarbamatherbizide, Triazinherbizide, Triazinonherbizide, Triazolherbizide, Triazolonherbizide, Triazolopyrimidinherbizide, Uracilherbizide, Ureaherbizide wie Phenylharnstoffherbizide oder Sulfonylharnstoffherbizide.

Bevorzugte Insektizide und Akarizide sind Carbamatinsektizide, wie Benzofuranylmethylcarbamat- Insektizide oder Dimethylcarbamat-Insektizide oder Oximcarbamat-Insektizide oder Phenylmethylcarbamatinsektizide, Diamidinsektizide, Insektenwachstumsregulatoren, Macrozyklische Lactoneinsektizide wie Avermectin-Insektizide oder Milbemycin-Insektizide oder Spinosyninsektizide, Nereistoxin analoge Insektizide, Nicotinoidinsektizide wie Nitroguanidinnicotinoid-Insektizide oder Pyridylmethylaminnicotinoid-Insektizide, Organophosphorinsektizide wie Organophosphatinsektizide oder Organothiophosphatinsektizide oder Phosphonatinsektizide oder Phosphoramidothioatinsektizide, Oxadiazininsektizide, Pyrazolinsektizide, Pyrethroidinsektizide wie Pyrethroidester-Insektizide oder Pyrethroidether-Insektizide oder Pyrethroidoxim-Insektizide, Tetramsäureinsektizide, Tetrahydrofurandioninsektizide, Thiazolinsektizide.

Als Beispiele für Fungizide seien genannt:
(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid, N-[1-(2,4-Dichlorphenyl)-1-methoxypropan-2-yl]-3-(difluormethyl)-1-methyl-1H-pyrazol-4-carboxamid, 5,8-Difluor-N-[2-(2-fluor-4-{[4-(trifluormethyl)pyridin-2-yl]oxy}phenyl)ethyl]quinazolin-4-amin, N-[9-(Dichlormethylen)-1,2,3,4-tetrahydro-1,4-methanonaphthalen-5-yl]-3-(difluormethyl)-1-methyl-1H-pyrazol-4-carboxamid, N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Coumethoxystrobin, Coumoxystrobin, Dimoxystrobin, Enestroburin, Famoxadon, Fenamidon, Fenoxystrobin, Fluoxastrobin, Kresoxim-Methyl, Metominostrobin, Orysastrobin, Picoxystrobin, Pyraclostrobin, Pyrametostrobin, Pyraoxystrobin, Pyribencarb, Triclopyricarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Meth6xy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl}-2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim, Pyrimethanil und 3-(5-Fluor-3,3,4,4-tetramethyl-3,4-dihydroisoquinolin-1-yl)quinolin.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, lodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon, Tricyclazol und 2,2,2-Trifluorethyl {3-methyl-1-[(4-methylbenzoyl)amino]butan-2-yl}carbamat.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl und Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Pyriofenon (Chlazafenon), Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrimorph, (2E)-3-(4-Tert-butylphenyl)-3-(2-chlorpyridin-4-yl)-1-(morpholin-4-yl)prop-2-en-1-on, (2Z)-3-(4-Tert-butylphenyl)-3-(2-chlorpyridin-4-yl)-1-(morpholin-4-yl)prop-2-en-1-on, Pyrrolnitrin, Tebufloquin, Tecloftalam, Tolnifanid, Triazoxid, Trichlamid, Zarilamid, (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2,6-Dimethyl-1H,5H-[1,4]dithiino[2,3-c:5,6-c']dipyrrol-1,3,5,7(2H,6H)-tetron, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3-(4,4,5-Trifluor-3,3-dimethyl-3,4-dihydroisoquinolin-1-yl)quinolin, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Fluor-2-[(4-fluorbenzyl)oxy]pyrimidin-4-amin, 5-Fluor-2-[(4-methylbenzyl)oxy]pyrimidin-4-amin, 5-Methyl-6-octyl[1,2,4]triazolo[1,5-a]pyrimidin-7-amin, Ethyl-(2Z)-3-amino-2-cyan-3-phenylprop-2-enoat, N'-(4-{[3-(4-Chlorbenzyl)-1,2,4-thiadiazol-5-yl]oxy}-2,5-dimethylphenyl)-N-ethyl-N-methylimidoformamid, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropylmethoxy)imino][6-(difluormethoxy)-2,3-difluorphenyl]methyl}-2-phenylacetamid, N'-{4-[(3-Tert-butyl-4-cyano-1,2-thiazol-5-yl)oxy]-2-chlor-5-methylphenyl}-N-ethyl-N-methylimidoformamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol, Chinolin-8-olsulfat(2:1) und Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon, N-[2-(4-{[3-(4-Chlorphenyl)prop-2-in-1-yl]oxy}-3-methoxyphenyl)ethyl]-N2-(methylsulfonyl)valinamid, 4-Oxo-4-[(2-phenylethyl)amino]butansäure und But-3-yn-1-yl{6-[({[(Z)-(1-methyl-1H-tetrazol-5-yl)(phenyl)methylen]amino}oxy)methyl]pyridin-2-yl}carbamat.

Als Beispiele für Bakterizide seien genannt:
Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

Als Beispiele für Insektizide, Akarizide und Nematizide seien genannt:
(1) Acetylcholinesterase (AChE) Inhibitoren, wie Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder Organophosphate, z.B. Acephate, Azamethiphos, Azinphos-ethyl, Azinphosmethyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodienorganochlorine, z.B. Chlordane und Endosulfan; oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)-Isomere)], Tralomethrin und Transfluthrin; oder DDT; oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam; oder Nikotin.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene; oder Fenoxycarb; oder Pyriproxyfen.
(8) Wirkstoffe mit unbekannten oder nicht spezifischen Wirkmechanismen, wie beispielsweise Alkylhalide, z.B. Methylbromid und andere Alkylhalide; oder Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
   Organozinnverbindungen, z.B. Azocyclotin, Cyhexatin und Fenbutatin-oxid; oder Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartap-hydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad; oder Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid; oder Cyanid.
(25) Komplex-II-Elektronentransportinhibitoren, wie beispielsweise Cyenopyrafen.
(28) Ryanodinrezeptor-Effektoren, wie beispielsweise Diamide, z.B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Pyridalyl, Pyrifluquinazon und lodmethan; sowie des weiteren Präparate auf Basis von Bacillus firmus (I-1582, BioNeem, Votivo).

Als Beispiele für Herbizide seien genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminocyclopyrachlor-kalium, Aminocyclopyrachlor-methyl, Aminopyralid, Amitrole, Ammoniumsulfamat, Ancymidol, Anilofos, Asulam, Atrazine, Aviglycin, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuronmethyl, Bentazone, Benzfendizone, Benzobicyclon, Benzofenap, Benzofluor, Benzoylprop, Benzyladenin, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbaryl, Carbetamide, Carfentrazone, Carfentrazone-ethyl, Carvone, Chlorcholinchlorid, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, 4-Chlorophenoxyacetic acid, Chlorophthalim, Chlorpropham, Chlorthal-dimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cloxyfonac, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, Cytokinine, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Diaminozid, Dicamba, Dichlobenil, Dichlorprop, Dichlorprop-P, Diclofop, Diclofopmethyl, Diclofop-P-methyl, Diclosulam, Diethatyl, Diethatyl-ethyl, Difenoxuron, Difenzoquat, Diflufenican, Diflufenzopyr, Diflufenzopyr-natrium, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Diisopropylnaphthalene, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethylnaphthylacetat, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl) pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-natrium, Glyphosate, Glyphosate-isopropylammonium, H-9201, d. h. O-(2,4-Dimethyl-6-nitrophenyl)-O-ethyl-isopropylphosphor amidothioat, Halosafen, Halosulfuron, Halosulfuron-methyl, Haloxyfop, Haloxyfop-P, Haloxyfopethoxyethyl, Haloxyfop-P-ethoxyethyl, Haloxyfop-methyl, Haloxyfop-P-methyl, Hexazinone, HW-02, d. h. 1-(Dimethoxyphosphoryl)-ethyl-(2,4-dichlorphenoxy)acetat, Imazamethabenz, Imazamethabenz-methyl, Imazamox, Imazamox-ammonium, Imazapic, Imazapyr, Imazapyr-isopropylammonium, Imazaquin, Imazaquin-ammonium, Imazethapyr, Imazethapyr-ammonium, Imazosulfuron, Inabenfide, Indanofan, Indaziflam, Indolessigsäure (IAA), 4-Indol-3-ylbuttersäure (IBA), lodosulfuron, lodosulfuron-methyl-natrium, lofensulfuron, lofensulfuron-natrium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuronmethyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, 1-Naphtylacetic Acid (NAA), Naphthylacetamid (NAAm), 2-Naphtoxyacetic Acid, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitroguaiacolate, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenzisopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobacnatrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosate-trimesium), Sulfosulfuron, SW-065, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triafamone, Triallate, Triasulfuron, Triaziflam, Triazofenamide, Tribenuron, Tribenuron-methyl, Tribufos, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P und Vernolate, ZJ-0862, d. h. 3,4-Dichlor-N-{2-[(4,6-dimethoxypyrimidin-2-yl)oxy]benzyl}anilin.

Als Beispiele für Pflanzenwuchsregulatoren seien ferner genannt natürliche Pflanzenhormone wie Abszissinsäure, Jasmonsäure, Salicylsäure bzw. deren Ester, Kinetin und Brassinosteroide.

Als Beispiele für Pflanzennährstoffe seien übliche anorganische oder organische Dünger zur Versorgung von Pflanzen mit Makro- und/oder Mikronährstoffen genannt.

Als Beispiele für Repellents seien Diethyltolylamid, Ethylhexandiol und Butopyronoxyl genannt.

Unter "Pflanzenschutzmittel" wird erfindungsgemäß die Anwendungsform der agrochemischen Wirkstoffe verstanden, z.B. Formulierungen und die Spritzbrühe.

Die Erfindung betrifft weiterhin Pflanzenschutzmittel sowohl in Form der handelsüblichen Formulierungen als auch den daraus bereitete Anwendungsformen wie Drench-, Drip- und Spritzbrühen, umfassend mindestens ein verethertes Tributylphenolalkoxylat der Formel (I) und einen oder mehrere agrochemische Wirkstoffe.

Gegebenenfalls enthalten die Anwendungsformen weitere Pflanzenschutzmittel und/oder die Wirkung verbessernde Adjuvantien wie Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe beispielsweise weitere Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind die erfindungsgemäßen veretherten Tributylphenolalkoxylate als Mittel, welche die Retention, das Anhaften an der Blattoberfläche und die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können weitere Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nichtaromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können auch geeigneten feste Trägerstoffe eingesetzt werden.

Auch verflüssigte gasförmige Streckmittel oder Lösemittel können grundsätzlich eingesetzt werden.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Stabilisatoren, Sequestiermittel, Komplexbildner, Humectans, Spreitmittel. Im Allgemeinen können die veretherten Tributylphenolalkoxylate der Formel (I) mit jedem festen oder flüssigen Zusatzstoff, welcher für Formulierungszwecke gewöhnlich verwendet wird, kombiniert werden.

Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% agrochemischen Wirkstoff oder, besonders bevorzugt zwischen 0,01 und 95 Gew.-% agrochemischen Wirkstoff, besonders bevorzugt zwischen 0,5 und 90 Gew.-% agrochemischen Wirkstoff, bezogen auf das Gewicht der Formulierung.

Der agrochemische Wirkstoffgehalt der aus dien Formulierungen bereiteten Anwendungsformen kann in weiten Bereichen variieren. Die Wirkstoffkonzentration der Anwendungsformen kann üblicherweise zwischen 0,00000001 und 95 Gew.-% agrochemischen Wirkstoffs, vorzugsweise zwischen 0,00001 und 1 Gew.-%, bezogen auf das Gewicht der Anwendungsform, liegen. Die Anwendung geschieht in einer den Anwendungsformen angepassten üblichen Weise.

Der Gehalt an den einzelnen Komponenten kann in den erfindungsgemäßen Formulierungen innerhalb eines größeren Bereichs variiert werden.

Die Herstellung der erfindungsgemäßen Formulierungen erfolgt z.B. in der Weise, dass man die Komponenten, einschließlich der veretherten Tributylphenolalkoxylate der Formel (I) in den jeweils gewünschten Verhältnissen miteinander vermischt. Handelt es sich bei dem agrochemischen Wirkstoff um eine Festsubstanz, so setzt man diesen im Allgemeinen entweder in fein gemahlener Form oder in Form einer Lösung oder Suspension in einem organischen Solvens oder Wasser ein. Ist der agrochemische Wirkstoff flüssig, so erübrigt sich häufig die Verwendung eines organischen Lösungsmittels. Es ist außerdem möglich, einen festen agrochemischen Wirkstoff in Form einer Schmelze einzusetzen.

Die Temperaturen können bei der Durchführung des Verfahrens in einem bestimmten Bereich variiert werden. Man arbeitet im Allgemeinen bei Temperaturen zwischen 0°C und 80°C, vorzugsweise zwischen 10°C und 60°C.

Bei der Durchführung des Verfahrens geht man im Allgemeinen so vor, dass man die veretherten Tributylphenolalkoxylate der Formel (I) mit einem oder mehreren Wirkstoffen sowie gegebenenfalls mit Zusatzstoffen vermischt. Die Reihenfolge, in der die Komponenten miteinander vermischt werden, ist beliebig. Zur Durchführung des Verfahrens kommen übliche Geräte in Betracht, die zur Herstellung von agrochemischen Formulierungen eingesetzt werden.

Als Applikationsformen können alle, dem Fachmann als gebräuchlich bekannten Verfahren verwendet werden; beispielsweise genannt seien: Spritzen, Tauchen, Nebeln sowie eine Reihe spezieller Verfahren zur direkten unter- oder oberirdischen Behandlung von gesamten Pflanzen oder Teilen (Saatgut, Wurzel, Stolonen, Stängel, Stamm, Blatt), wie beispielsweise Stamminjektion bei Bäumen oder Stängelbandagen bei perennierenden Pflanzen, und eine Reihe spezieller indirekter Applikationsverfahren.

Die jeweilige flächen- und/oder objektbezogene Aufwandmenge der Pflanzenschutzmittel unterschiedlichster Formulierungstypen zur Bekämpfung der genannten Schadorganismen variiert sehr stark. Im Allgemeinen werden hierfür die, dem Fachmann als gebräuchlich für das jeweilige Einsatzgebiet bekannten Applikationsmedien in den gebräuchlichen Mengen eingesetzt; wie beispielsweise von mehreren hundert Liter Wasser pro Hektar bei Standard-Spritzverfahren über wenige Liter Öl pro Hektar bei der 'Ultra Low Volume'-Flugzeugapplikation bis hin zu wenigen Millilitern einer physiologischen Lösungen bei Injektionsverfahren. Die Konzentrationen der erfindungsgemäßen Pflanzenschutzmittel in den entsprechenden Applikationsmedien variieren daher in einem weiten Bereich und sind vom jeweiligen Einsatzgebiet abhängig. Im Allgemeinen werden Konzentrationen verwendet, die dem Fachmann als gebräuchlich für das jeweilige Einsatzgebiet bekannt sind. Bevorzugt sind Konzentrationen von 0,01 Gew.% bis 99 Gew.%, besonders bevorzugt von 0,1 Gew.% bis 90 Gew.%.

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden.

Bevorzugt sind Pflanzen aus der Gruppe der Nutzpflanzen, Zierpflanzen, Rasenarten, allgemein genutzte Bäume, die in öffentlichen und privaten Bereichen als Zierpflanzen Verwendungen finden, und Forstbestand. Der Forstbestand umfasst Bäume für die Herstellung von Holz, Zellstoff, Papier und Produkten, die aus Teilen der Bäume hergestellt werden.

Der Begriff Nutzpflanzen, wie hier verwendet, bezeichnet Kulturpflanzen, die als Pflanzen für die Gewinnung von Nahrungsmitteln, Futtermitteln, Treibstoffen oder für technische Zwecke eingesetzt werden.

Die Erfindung wird durch die Beispiele näher erläutert, ohne sie darauf zu beschränken.

### Beispiele

Herstellung von 2,4,6-Tri-sec-butylphenolethoxylat-methylethern

### Beispiel 1

Herstellung von 2,4,6-Tributylphenoxy-ethoxy(6)-methylether (TBPEM-6) 563 g Sapogenat® T 060 (Tributylphenolethoxylat(6)) (Clariant) wurden unter Stickstoffatmosphäre auf 60 °C temperiert und mit 60 g fester NaOH versetzt. Danach wurde evakuiert und über einen Zeitraum von 3 h 75,7 g MeCl eindosiert, wobei es zu einer leichten Erwärmung um ca. 5 °C kam. Nach erfolgter Zugabe wurde mindestens 3 h bei 80 °C gerührt, danach Reaktionswasser und überschüssiges MeCI durch langsame Druckreduktion entfernt. Je nach Umsatzgrad war das beschriebene Prozedere zu wiederholen.
Nach Erreichen des geforderten Umsatzes wurde Wasser zugegeben und mindestens 1 h bei 80 °C gerührt, nachfolgend wurde der Ansatz zur Phasentrennung stehen gelassen. Die organische Phase wurde mit Phosphorsäure über einen Zeitraum von 30 min neutralisiert, entwässert und über Celite filtriert. Die Ausbeute an TBPEM-6 betrug 577 g.
Analog wurde das methylveretherte Tributylphenolalkoxylat TBPEM-8 aus dem mit acht Mol Ethylenoxid ethoxylierten Sapogenat T 080 erhalten.

### Beispiel 2

### Grenzflächenaktivität - Netzmittelwirkung

Die dynamische Oberflächenspannung wurde über die Blasendruckmethode bestimmt (Tensiometer BP2100, Krüss) auf einer Zeitskala, die für die Spritzapplikation relevant ist. Die dynamische Oberflächenspannung würde für Lösungen von TBPEM-6 und TBPEM-8 in Leitungswasser gemessen bei Raumtemperatur (22°C) und den typischen Spritzbrühenkonzentrationen von 0.3, 1 und 3 g/l.
Die niedrigen dynamischen Oberflächenspannungen, insbesondere von TBPEM-6, zeigen die exzellente Eignung als schnelles Netzmittel inklusive die Eignung als Haftförderer bei der Spritzapplikation von Pflanzenschutzmitteln. Im mittleren Zeitbereich von 100 Millisekunden war dabei die Netzwirkung besser

| | Dynamische | Oberflächenspannung [mN/m] | | |
|---|---|---|---|---|
| Substanz | Konz [g/L] | 20ms | 100ms | 200ms |
| Sapogenat T 060 | 0.3 | 69.9 | 64.6 | 61.4 |
| Sapogenat T 060 | 1 | 67.3 | 58.6 | 53.2 |
| Sapogenat T 060 | 3 | 63.4 | 51.8 | 46 |
| TBPEM-6 | 0.3 | 70.3 | 65.0 | 62.8 |
| TBPEM-6 | 1 | 62.9 | 54.1 | 52.5 |
| TBPEM-6 | 3 | 55.5 | 42.9 | 41.8 |
| | | | | |
| Sapogenat T 080 | 0.3 | 69.8 | 63.2 | 60.3 |
| Sapogenat T 080 | 1 | 64.7 | 57.3 | 52.2 |
| Sapogenat T 080 | 3 | 62.3 | 51.3 | 45.2 |
| TBPEM-8 | 0.3 | 69.1 | 61.7 | 58.6 |
| TBPEM-8 | 1 | 63.3 | 56.2 | 50 |
| TBPEM-8 | 3 | 59 | 49.7 | 44.6 |

### Beispiel 3

### Prozentuale Penetration von Benzyladenin durch Apfelkutikeln mit und ohne TBPEM-6

Die Penetration des Wachstumsförderers Benzyladenin (Benzylamonopurin) durch Blattkutikeln wurde bei einer Wirkstoffkonzentration von 0.2 g/l Benzyladenin in Aceton/Leitungswasser gemessen mit und ohne TBPEM-6 bei verschiedenen praxisrelevanten Konzentrationen 5 Stunden und 24 Stunden nach Applikation.

Im Vergleich zu Benzyladenin gelöst in Aceton/Leitungswasser wurde bei Zusatz von TBPEM-6 eine mehr als siebenfach höhere Penetration, gemessen nach 5 und 24 Stunden, erzielt. TBPEM-6 wurde in einer typischen Adjuvantienkonzentration von 0,5, 1,0 und 3,0 g/l zugesetzt:

| Benzyladenin + | 5h | 24h |
|---|---|---|
| Kontrolle (reiner Wirkstoff) | 2.0 | 3.7 |
| 0. 5 g/l TBPEM-6 | 18.0 | 26.1 |
| 1.0 g/l TBPEM-6 | 18.6 | 27.5 |
| 3.0 g/l TBPEM-6 | 45.5 | 58.9 |

### Beispiel 4.

Die Penetration des Herbizids Atrazin durch Blattkutikeln wurde bei einer Wirkstoffkonzentration von 5 g/l Atrazin (SC500) gemessen mit und ohne dem kommerziellen Additiv Aureo® (Rapsölmethylester) bei 2.5 g/l bei 1 g/l für die Zeitpunkte 6, 18 und 48 Stunden nach Applikation.

Im Vergleich zur Atrazin SC-Formulierung allein wurde zu allen drei Zeitpunkten eine mindestens vierfach bis sechsfach höhere Penetration gemessen, wenn bei 1 g/l zugesetzt wurde. Die Penetration von Atrazin war sogar höher als die mit der 2.5-fach höheren Konzentration des kommerziellen Standards Aureo.

| Atrazin SC500 | g/l | 6h | 18h | 48h |
|---|---|---|---|---|
| Kontrolle (reiner Wirkstoff) | | 0.4 | 1.0 | 4.8 |
| + Aureo | 2.5 | 1.8 | 4.5 | 9.8 |
| + TBPEM-6 | 1 | 1.9 | 6.7 | 17.9 |

## Patentansprüche

1. Veretherte Tributylphenolalkoxylate der Fomel (I), 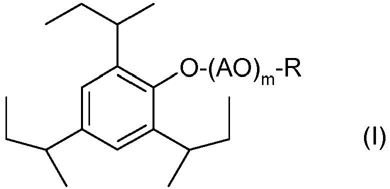 worin
R für Methyl steht, und
A für einen Ethylen-Rest, einen 1,2-Propylen-Rest, einen 1,2-Butylen-Rest oder für Gemische aus Ethylen- und 1,2-Propylen-Resten oder für Gemische aus 1,2-Ethylen- und 1,2-Butylen-Resten steht und
m für eine Zahl von 2 bis 30 steht.

2. Veretherte Tributylphenolalkoxylate nach Anspruch 1, worin
R für Methyl steht,
A für einen 1,2-Ethylen-Rest, einen 1,2-Propylen-Rest oder für Gemische aus 1,2-Ethylen- und 1,2-Propylen-Resten steht und
m für eine Zahl von 2 bis 20 steht.

3. Veretherte Tributylphenolalkoxylate nach Anspruch 1 oder 2, worin
R für Methyl steht,
A für einen Ethylen-Rest steht und
m für eine Zahl von 3 bis 10 steht.

4. Veretherte Tributylphenolalkoxylate nach einem der Ansprüche 1 bis 3, worin m für eine Zahl von 5 bis 8 steht.

5. Verfahren zur Herstellung der veretherten Tributylphenolalkoxylate nach einem der Ansprüche 1 bis 4, umfassend den Schritt
a) Umsetzung eines Tributylphenolalkoxylats der Formel (II), 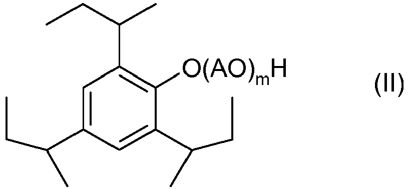 worin A und m die in einem der Ansprüche 1 bis 4 angegebenen Bedeutungen haben, mit einem C₁-Alkylhalogenid unter Basenkatalyse.

6. Verwendung der veretherten Tributylphenolalkoxylate der Formel (I) nach einem der Ansprüche 1 bis 4 in Pflanzenschutzmitteln.

7. Verwendung der veretherten Tributylphenolalkoxylate der Formel (I) nach einem der Ansprüche 1 bis 4 zur Verbesserung der Wirkung von agrochemischen Wirkstoffen in und auf Pflanzen.

8. Verwendung nach Anspruch 7 zur Verbesserung der Retention oder der Blattaufnahme von agrochemischen Wirkstoffen.

9. Verwendung der veretherten Tributylphenolalkoxylate der Formel (I) nach einem der Ansprüche 1 bis 4 als Tensid, als Netz und Haftmittel oder als Emulgator.

10. Pflanzenschutzmittel, enthaltend
A) einen oder mehrere agrochemische Wirkstoffe und
B) ein oder mehrere veretherten Tributylphenolalkoxylate der Formel (I) gemäß einem der Ansprüche 1 bis 4.

## Claims

1. Etherified tributylphenol alkoxylate of the formula (I), 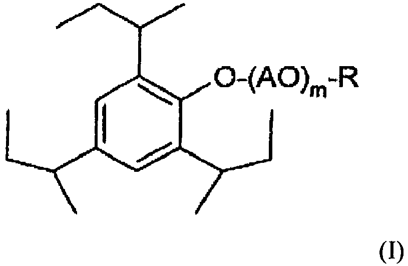 wherein
R is methyl, and
A is an ethylene radical, a 1,2-propylene radical, a 1,2-butylene radical, or mixtures of ethylene and 1,2-propylene radicals or mixtures of 1,2-ethylene and 1,2-butylene radicals, and
m is a number from 2 to 30.

2. Etherified tributylphenol alkoxylate according to Claim 1, in which
R is methyl,
A is a 1,2-ethylene radical, a 1,2-propylene radical, or mixtures of 1,2-ethylene and 1,2-propylene radicals, and
m is a number from 2 to 20.

3. Etherified tributylphenol alkoxylate according to Claim 1 or 2, in which
R is methyl,
A is an ethylene radical, and
m is a number from 3 to 10.

4. Etherified tributylphenol alkoxylate according to any of Claims 1 to 3, in which m is a number from 5 to 8.

5. Process for preparing an etherified tributylphenol alkoxylate according to any of Claims 1 to 4, comprising the step of
a) reacting a tributylphenol alkoxylate of the formula (II), 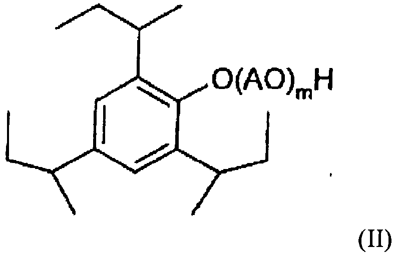 in which A and m have the definitions indicated in any of Claims 1 to 4, with a C₁- alkyl halide, with catalysis by base.

6. Use of an etherified tributylphenol alkoxylate of the formula (I) according to any of Claims 1 to 4 in a crop protection composition.

7. Use of an etherified tributylphenol alkoxylate of the formula (I) according to any of Claims 1 to 4 for improving the effect of active agrochemical ingredients in and on plants.

8. Use according to Claim 7 for improving the retention or the foliar uptake of active agrochemical ingredients.

9. Use of an etherified tributylphenol alkoxylate of the formula (I) according to any of Claims 1, to 4 as surfactant, as wetter and sticker, or as emulsifier.

10. Crop protection composition comprising
A) one or more active agrochemical ingredients and
B) one or more etherified tributylphenol alkoxylates of the formula (I) according to any of Claims 1 to 4.

## Revendications

1. Alcoxylates de tributylphénol éthérifiés de formule (I) 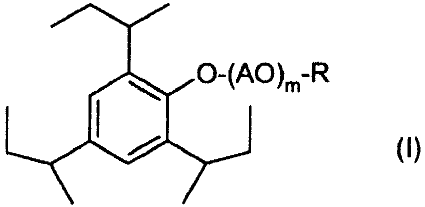 dans lesquels
R représente méthyle et
A représente un radical éthylène, un radical 1,2-propylène, un radical 1,2-butylène ou des mélanges de radicaux éthylène et 1,2-propylène ou des mélanges de radicaux 1,2-éthylène et 1,2-butylène, et
m représente un nombre de 2 à 30.

2. Alcoxylates de tributylphénol éthérifiés selon la revendication 1, dans lesquels
R représente méthyle et
A représente un radical 1,2-éthylène, un radical 1,2-propylène ou des mélanges de radicaux 1,2-éthylène et 1,2-propylène, et
m représente un nombre de 2 à 20.

3. Alcoxylates de tributylphénol éthérifiés selon la revendication 1 ou 2, dans lesquels
R représente méthyle et
A représente un radical éthylène, et
m représente un nombre de 3 à 10.

4. Alcoxylates de tributylphénol éthérifiés selon l'une quelconque des revendications 1 à 3 dans lesquels m représente un nombre de 5 à 8.

5. Procédé de fabrication des alcoxylates de tributylphénol éthérifiés selon l'une quelconque des revendications 1 à 4, comprenant l'étape suivante :
a) la mise en réaction d'un alcoxylate de tributylphénol de formule (II) 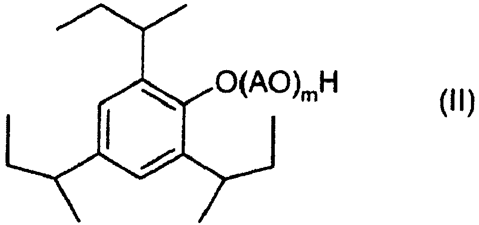 dans laquelle A et m ont les significations indiquées dans l'une quelconque des revendications 1 à 4, avec un halogénure d'alkyle en C₁ sous catalyse basique.

6. Utilisation des alcoxylates de tributylphénol éthérifiés de formule (I) selon l'une quelconque des revendications 1 à 4 dans des agents phytosanitaires.

7. Utilisation des alcoxylates de tributylphénol éthérifiés de formule (I) selon l'une quelconque des revendications 1 à 4 pour améliorer l'effet d'agents actifs agrochimiques dans et sur des plantes.

8. Utilisation selon la revendication 7 pour améliorer la rétention ou l'absorption par les feuilles d'agents actifs agrochimiques.

9. Utilisation des alcoxylates de tributylphénol éthérifiés de formule (I) selon l'une quelconque des revendications 1 à 4 en tant que tensioactif, en tant qu'agent mouillant et adhésif ou en tant qu'émulsifiant.

10. Agent phytosanitaire, contenant :
A) un ou plusieurs agents actifs agrochimiques et
B) un ou plusieurs alcoxylates de tributylphénol éthérifiés de formule (I) selon l'une quelconque des revendications 1 à 4.
